(19) Europäisches Patentamt
European Patent Office
Office européen des brevets

(11) EP 0 573 644 B1

(12) FASCICULE DE BREVET EUROPEEN

(45) Date de publication et mention de la délivrance du brevet: **31.01.1996 Bulletin 1996/05**

(21) Numéro de dépôt: **93902290.1**

(22) Date de dépôt: **03.12.1992**

(51) Int Cl.6: **C07C 323/12**, C07C 43/13, A61K 7/00, C07C 317/18

(86) Numéro de dépôt international: **PCT/FR92/01140**

(87) Numéro de publication internationale: **WO 93/11103 (10.06.1993 Gazette 1993/14)**

# (54) COMPOSES HYDRO-FLUORO-CARBONES, LEUR UTILISATION DANS DES COMPOSITIONS COSMETIQUES, LEUR PROCEDE DE PREPARATION ET LES COMPOSITIONS COSMETIQUES LES COMPORTANT

FLUORIERTE KOHLENWASSERSTOFFDERIVATE, IHRE VERWENDUNG IN KOSMETISCHEN ZUSAMMENSETZUNGEN, VERFAHREN ZU IHRER HERSTELLUNG UND SIE ENTHALTENDE KOSMETISCHE ZUSAMMENSETZUNGEN

FLUORINATED HYDROCARBON COMPOUNDS, THEIR USE IN COSMETIC COMPOSITIONS, METHOD OF PREPARING THEM AND COSMETIC COMPOSITIONS CONTAINING THEM

(84) Etats contractants désignés:
**AT BE CH DE DK ES FR GB GR IT LI NL SE**

(30) Priorité: **04.12.1991 FR 9115019**

(43) Date de publication de la demande: **15.12.1993 Bulletin 1993/50**

(73) Titulaire: **L'OREAL**
**F-75008 Paris (FR)**

(72) Inventeurs:
- **MAHIEU, Claude**
  **F-75017 Paris (FR)**
- **BOLLENS, Eric**
  **F-94410 Saint-Maurice (FR)**
- **MELLUL, Myriam**
  **F-94240 L'Hay-les-Roses (FR)**

(74) Mandataire: **Casalonga, Axel et al**
**D-80469 München (DE)**

(56) Documents cités:
**DE-A- 2 052 579** **FR-A- 2 416 222**
**FR-A- 2 516 920** **US-A- 4 126 702**

- **CHEMICAL ABSTRACTS, vol. 110, no. 16, 17 Avril 1989, Columbus, Ohio, US; abstract no. 141248p, H. MATSUO ET AL. 'Cosmetic skin and hair preparations containing fluoroalkyl-containing ethers.' page 386 ;colonne 2 ; & JP-A-63 183 508**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1)Convention sur le brevet européen).

EP 0 573 644 B1

Printed by Jouve, 75001 PARIS (FR)

## Description

La présente invention concerne des composés hydro- et fluoro-carbonés utiles notamment dans des compositions cosmétiques, et leur procédé de préparation ainsi que des compositions cosmétiques comportant ces composés.

Le document US 4 126 702 décrit certains dérivés du glycérol ainsi que leur application dans des compositions cosmétiques.

Il est connu d'utiliser des perfluoropolyéthers, notamment dans des procédés de nettoyage, de protection, de maquillage de la peau ou bien de lavage des cheveux. Ces composés sont connus pour leur basse tension superficielle et leur facilité d'étalement, mais présentent une solubilité dans la plupart des fluides très réduite, excepté dans les fluides fluorés, ce qui rend très difficile leur formulation dans des compositions cosmétiques. Certains de ces composés, les perfluorométhylisopropyléthers, sont connus sous le nom de "FOMBLIN HC", commercialisés par la Société MONTEFLUO.

La demanderesse a maintenant découvert des composés nouveaux qui, contrairement aux FOMBLIN connus, présentent une bonne solubilité, notamment dans les solvants classiques utilisés en cosmétique comme les alcools inférieurs, ainsi que les corps gras et huiles usuels. Leur caractère amphiphile, leurs propriétés et leur compatibilité avec les solvants permettent notamment de préparer des compositions homogènes et stables, et par exemple d'assurer une bonne stabilité des émulsions dans laquelle ils interviennent.

Ainsi, la présente invention concerne les composés de formule :

$$R_F - (CH_2)_n - X - [C_3H_5(OH)] - (Y)_x - R_H \qquad (I)$$

dans laquelle $C_3H_5(OH)$ représente les structures :

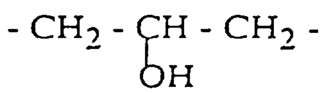 ou 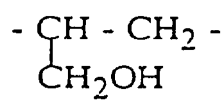

(Ia) (Ib)

$R_F$ représente un radical alkyle perfluoré en $C_4$-$C_{20}$ ou un mélange de radicaux alkyle perfluorés en $C_4$-$C_{20}$;

$R_H$ représente un radical alkyle linéaire ou ramifié en $C_1$-$C_{22}$ ou un mélange de radicaux alkyle linéaires ou ramifiés en $C_1$-$C_{22}$ ou un radical aryle ou aralkyle;

n est compris entre 0 et 4;

X représente O, S ,

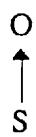 ou 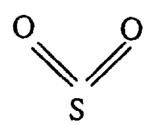 ;

x représente O ou 1 ;

Y représente O, S,

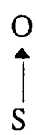 ou 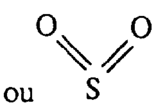 ;

sous réserve que lorsque X = S,

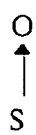 ou 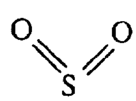 ,

Y n'est pas S,

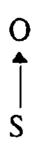 ou 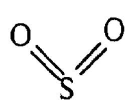 .

Parmi ces composés, les composés préférés sont ceux pour lesquels $R_F$ désigne un radical alkyle perfluoré en $C_6$-$C_{12}$, $R_H$ désigne un radical alkyle linéaire ou ramifié en $C_3$-$C_{18}$, un radical aryle en $C_6$-$C_{10}$ ou un radical aralkyle en $C_7$-$C_{15}$ et n est 2.

De préférence, X est O ou S et Y est O.

Parmi les radicaux alkyle linéaires ou ramifiés, on peut citer notamment les radicaux butyle, octyle, 2-éthylhexyle, décyle, dodécyle, tétradécyle, hexadécyle, 2-hexyldécyle, stéaryle et isostéaryle.

Parmi les radicaux aralkyle, on peut citer notamment les radicaux 4-nonylphényle et benzyle, et parmi les radicaux aryle, le radical phényle.

Les composés de formule (I) selon l'invention peuvent être préparés en mettant en oeuvre la réaction d'un composé fluoré à hydrogène acide de formule (II) :

$$R_F - (CH_2)_n - X - H \qquad (II)$$

avec un époxyde de formule (III) :

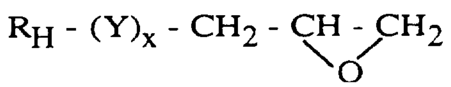

(III)

ou la réaction d'un composé hydrocarboné à hydrogène acide de formule (IV) :

$$R_H - (Y)_x - H \qquad (IV)$$

avec un époxyde fluoré de formule (V) :

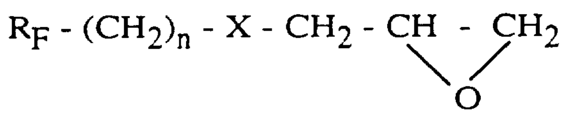

(V)

en présence d'un composé basique ou acide jouant le rôle de réactif ou de catalyseur, pour obtenir le composé de formule (I) correspondant, les substituants $R_F$, $R_H$, n et x ayant dans les formules (II) et (III), (IV) et (V), la même signification que dans la formule (I) et X désignant O ou S, Y désignant O ou S, sous réserve que lorsque X est S, Y n'est pas S, et en oxydant éventuellement la fonction mercaptan en sulfoxyde ou sulfone avec de l'eau oxygénée.

Les composés de formule (V) sont décrits notamment dans le brevet US 3976698 ou dans les demandes de brevet EP 300358 et DE 2018461.

Lorsque X représente S dans la formule (II) ou (IV), on utilise de préférence un composé basique.

Les composés jouant le rôle de réactif ou de catalyseur peuvent donc être basiques, tels que les métaux alcalins, les hydroxydes de métaux alcalins ou alcalino-terreux, les alcoolates des métaux alcalins tels que les méthylates ou tertiobutylates, les hydrures alcalins tels que l'hydrure de sodium, les amines tertiaires telles que la pyridine ou la triéthylamine. Ils peuvent aussi être des bases de Lewis parmi lesquelles on peut citer les fluorures de césium, de rubidium, de potassium. Ces composés peuvent être également supportés sur un solide tel que l'alumine. De préférence, on utilise un alcoolate alcalin comme le méthylate de sodium ou une amine tertiaire comme la pyridine.

Ces composés peuvent également être acides, notamment lorsque le produit de départ de formule (II) ou (IV) est un alcool. De tels acides peuvent être les acides minéraux ou leurs sels avec des amines tertiaires ou encore des acides dits de Lewis tels que le trifluorure de bore, le tétrachlorure d'étain, le pentachlorure d'antimoine, utilisés seuls, en solution ou associés à un support usuel.

La concentration des composés acides ou basiques jouant le rôle de réactif ou de catalyseur et mis en oeuvre dans la réaction de préparation des composés de formule (I) peut être comprise entre 1 et 100% molaire, de préférence entre 2 et 10% molaire par rapport au composé fluoré à hydrogène acide de formule (II) ou (IV).

La réaction de préparation peut être mise en oeuvre en présence de solvant ou en l'absence de solvant.

Comme solvant, on peut utiliser des hydrocarbures aliphatiques tels que l'heptane, l'hexane ou des hydrocarbures cycliques tels que le cyclohexane, des hydrocarbures aromatiques tels que le toluène, des éthers tels que l'éther éthylique ou isopropylique, des éthers cycliques tels que le dioxanne, ou bien encore l'acétonitrile, le diméthylformamide, la N-méthylpyrrolidone, le diméthylacétamide.

Lorsque le composé de formule (II) ou (IV) est un thiol (X=S), les alcools tels que le méthanol, l'éthanol ou l'isopropanol peuvent aussi être utilisés comme solvant.

Pour préparer les composés selon l'invention, on peut mélanger d'abord le composé à hydrogène acide de formule (II) ou (IV) avec le réactif ou catalyseur acide ou basique, sous atmosphère inerte. Pour réaliser le mélange, on peut opérer à une température comprise entre 20 et 180°C, de préférence entre 50 et 150°C. Par atmosphère inerte, on entend par exemple une atmosphère d'azote, d'argon ou d'hélium.

Le mélange peut être effectué, selon la nature du composé à hydrogène acide et du réactif ou catalyseur, en l'absence ou en présence de solvant.

On ajoute au mélange obtenu l'époxyde de formule (III) ou (V), cette addition pouvant s'effectuer en une seule fois ou progressivement, sur une période pouvant aller de 30 minutes à 2 heures par exemple.

Le temps de réaction est alors compris entre environ 1 heure et 24 heures, de préférence entre 1 heure et 3 heures.

Le composé issu de la réaction peut être oxydé lorsque celui-ci contient une fonction mercaptan, en sulfoxyde ou en sulfone, en présence d'eau oxygénée en milieu acide, selon des méthodes connues, notamment dans les demandes de brevet français FR 2 099 092 et FR 2 516 920.

Il peut être en outre nécessaire de neutraliser le mélange obtenu, et on peut séparer le composé synthétisé de façon usuelle, par distillation, par exemple.

Lorsque la réaction de préparation des composés de formule (I) est effectuée en présence de composé basique, elle conduit uniquement à des composés pour lesquels $C_3H_5(OH)$ représente le groupement (Ia). Dans le cas où la réaction est effectuée en présence de composé acide, on peut obtenir un mélange de deux composés correspondant aux significations (Ia) et (Ib) de $C_3H_5(OH)$.

Les composés selon l'invention peuvent se présenter sous forme d'huile ou sous forme solide à la température ambiante.

Un autre objet de l'invention concerne l'utilisation des produits de formule (I), composés amphiphiles, notamment dans des compositions cosmétiques.

De façon générale, ces composés utilisés dans des compositions cosmétiques permettent d'en améliorer les propriétés cosmétiques. Ils confèrent de la douceur, de la brillance et un toucher non collant aux matières kératiniques telles que la peau, les cheveux et les ongles. Par ailleurs, certains de ces composés se présentant sous forme d'huile incolore, ils peuvent permettre d'obtenir des émulsions transparentes.

C'est pourquoi la présente invention concerne également des compositions cosmétiques caractérisées en ce qu'elles comportent au moins un composé de formule (I) définie ci-dessus.

Ces compositions peuvent se présenter sous forme d'émulsions, de laits ou de crèmes, de lotions huileuses ou oléoalcooliques, sous forme de gels gras ou oléoalcooliques, de dispersions vésiculaires à base de lipides amphiphiles ioniques ou non-ioniques, de bâtonnets solides, de pâte, de spray ou de mousse aérosol.

Selon la forme des compositions auxquelles sont incorporés les composés de l'invention, ces compositions comportent par ailleurs les adjuvants et additifs usuels pour la forme choisie.

Plus précisément, ces compositions peuvent être des laits et des crèmes pour les soins de la peau ou des cheveux, des crèmes, lotions ou laits démaquillants, des crèmes, gels, laits ou lotions anti-solaire, des crèmes ou mousses de rasage, des lotions après-rasage, des shampooings ou des après-shampooings, des déodorants corporels, des pâtes dentifrices, des laques, des produits de soin des lèvres ou des ongles.

Ces compositions cosmétiques peuvent être également utilisées comme produit de maquillage des cils, des sourcils, des ongles, des lèvres ou de la peau comme les crèmes de traitement de l'épiderme, des fonds de teint, des bâtons de rouge à lèvres, des fards à paupières ou à joues, des eye-liners ou mascara, des vernis à ongles, par exemple.

Selon l'invention, les composés de formule (I) représentent de 0,1 à 25%, et de préférence de 0,1 à 15% du poids total de la composition.

Parmi les adjuvants usuels pour ce genre de composition, peuvent être présents en outre dans les compositions selon l'invention, des corps gras usuels, des solvants organiques, des silicones, les épaississants, les adoucissants, des filtres solaires UV-A ou UV-B ou à bande large, des agents anti-mousses, des agents hydratants, des humectants, des parfums, des conservateurs, des tensio-actifs, des charges, des sequestrants, des émulsionnants, des polymères anioniques, cationiques, non-ioniques, amphotères ou leurs mélanges, des anti-perspirants, des agents alcalinisants, des colorants, des pigments, des agents propulseurs, des anti-oxydants et des anti-radicaux libres.

Plus précisément, comme corps gras, on peut utiliser une huile ou une cire ou leur mélange, des acides gras, des alcools gras, des esters d'acides gras tels que les triglycérides d'acides gras en $C_6$ à $C_{18}$, de la vaseline, de la paraffine, de la lanoline, de la lanoline hydrogénée ou acétylée.

Parmi les huiles, on peut citer les huiles minérales, animales, végétales ou les huiles de synthèse, et notamment l'huile de vaseline, de paraffine, de ricin, de jojoba, de sésame, ainsi que les huiles et des gommes de silicone et les isoparaffines.

Parmi les cires animales, fossiles, végétales, minérales ou de synthèse, on peut notamment citer les cires d'abeille, de Caroube, de Candelila, les ozokérites, les cires microcristallines ainsi que les cires et résines de silicone.

Parmi les solvants organiques usuellement utilisés dans les compositions cosmétiques, on peut citer plus précisément les mono-alcools ou polyalcools inférieurs en $C_1$ à $C_6$ comme l'éthanol, l'isopropanol, le propylèneglycol, le glycérol, le sorbitol, les cétones telles que l'acétone, les esters tels que l'acétate de butyle ou l'acétate d'éthyle, le toluène, par exemple.

A titre d'agent épaississant, on peut citer les dérivés de cellulose, les dérivés d'acide polyacrylique, les gommes de guar ou de caroube, ainsi que la gomme de xanthane, par exemple.

Parmi les tensio-actifs, on peut citer notamment les tensio-actifs non-ioniques tels que les alkyl($C_8$-$C_{24)}$polyglycosides où le nombre de motif glucoside est compris entre 1 et 15 et les tensio-actifs non-ioniques du type polyglycérolé.

Les alkylpolyglycosides sont notamment les produits vendus sous la dénomination APG, tels que les produits APG 300, APG 350, APG 500, APG 550, APG 625, APG base 10-12; les produits vendus par la Société SEPPIC sous les dénominations TRITON CG 110 et TRITON CG 312.

Les composés polyglycérolés sont les dérivés qui résultent de la condensation de 1 à 10, de préférence de 2 à 6 moles de glycidol par mole d'alcool ou d'alphadiol en $C_{10}$-$C_{14}$, de diglycolamide d'acides gras en $C_{12}$-$C_{18}$, tels que décrits dans les brevets FR 1 477 048, 2 328 763, 2 091 516, 2 169 787.

Les dispersions vésiculaires de lipides amphiphiles ioniques ou non-ioniques citées ci-dessus, peuvent être préparées selon des procédés usuels dont on trouve une liste non limitative dans "Les liposomes en biologie cellulaire et pharmacologie" Edition INSERM/John Libbery Eurotext, (1987), p. 6 à 18.

Pour les compositions sous forme de pâte dentifrice, on peut utiliser les adjuvants et additifs usuels comme des agents de polissage tels que la silice, des agents actifs comme les fluorures tels que le fluorure de sodium, et éventuellement des agents édulcorants tels que le saccharinate de sodium.

Les exemples suivants sont destinés à illustrer l'invention et ne sauraient être considérés comme limitatifs de sa portée.

**EXEMPLES DE PREPARATION**

EXEMPLE 1

**1-(2'-F-hexyléthylthiol 3-(2"-éthylhexyloxy)-2-propanol**

a) en présence de méthylate de sodium :

A la température de 25°C, sous agitation et sous courant d'azote, on ajoute à 152 g de 2-F-hexyléthanethiol, 3,6 g d'une solution méthanolique de méthylate de sodium (environ 30% - 5,54 meq $g^{-1}$) en une minute.

Le mélange est chauffé à 70°C. On évapore sous vide le méthanol présent dans le milieu.

Le 2-éthylhexylglycidyléther (74,4 g) est ensuite ajouté goutte-à-goutte en une heure. On maintient la température du mélange entre 60 et 70°C au cours de l'addition de l'époxyde.

A la fin de l'addition, la température est amenée à 25°C.

Le mélange est neutralisé à l'aide de 20 ml de HCl Normal.

Le 1-(2'-F-hexyléthylthio)3-(2"-éthylhexyloxy)2-propanol est séparé par distillation : Eb = 141°C/66,5 Pa.

On obtient 175 g (77%) d'une huile translucide incolore.

Analyse élémentaire :

| | %C | %H | %S | %F |
|---|---|---|---|---|
| Calculé | 40,28 | 4,80 | 5,66 | 43,60 |
| Mesuré | 40,37 | 4,82 | 5,55 | 43,74 |

b) en présence de fluorure de potassium déposé sur alumine :

Dans un réacteur de 1 litre, on introduit 304 g de 2-F-hexyléthanethiol. Sous courant d'azote, on ajoute 7,6 g de fluorure de potassium déposé sur alumine ($5,5.10^{-3}$ mole de $F^-$/g). En maintenant la température à 40°C, on additionne 148,8 g de 2-éthylhexylglycidyléther en 2 heures. Le mélange est laissé sous agitation à 25°C pendant 4 heures.

500 ml de dichlorométhane sont ajoutés ensuite au mélange réactionnel.

Le fluorure de potassium déposé sur alumine est alors éliminé par filtration sur verre fritté n°4. Après évaporation du dichlorométhane, le 1-(2'-F-hexyléthylthio)-3-(2"-éthylhexyloxy)-2-propanol est séparé par distillation sous pression réduite (148-150°C/6,65 Pa).

On obtient 340 g (75%) d'une huile incolore.

Analyse élémentaire :

| | %C | %H | %S | %F |
|---|---|---|---|---|
| Calculé | 40,28 | 4,80 | 5,66 | 43,60 |
| Mesuré | 39,94 | 4,75 | 5,38 | 43,76 |

EXEMPLE 2

**1 (2'-F-octyléthylthio)3-(2"-éthylhexyloxy)-2-propanol**

Le composé est préparé de façon analogue à la préparation décrite à l'exemple 1 où l'on utilise :

- 288 g de 2-F-octyléthanethiol
- 5,4 g d'une solution méthanolique de méthylate de sodium (5,54 meq $g^{-1}$)
- 111,6 g de 2-éthylhexylglycidyléther
- 30 ml de HCl normal

On obtient 337 g (81%) d'une huile translucide incolore.
Analyse élémentaire :

| | %C | %H | %S | %F |
|---|---|---|---|---|
| Calculé | 37,84 | 4,08 | 4,81 | 48,46 |
| Mesuré | 37,83 | 4,06 | 4,20 | 47,45 |

EXEMPLE 3

**1-(2'-F-octyléthylthio)3-butyloxy 2-propanol**

Selon le mode opératoire décrit à l'exemple 1, on condense en 1 heure 40 g (0,31 mole) d'éther de butyle et de glycidyle sur 147,7 g (0,31 mole) de 2-F-octyléthanethiol, en présence de 2,75 g de solution méthanolique de méthylate de sodium (5,54 meq $g^{-1}$). En fin de réaction, le mélange est neutralisé par 15,5 ml de HCl Normal.

Après distillation (138° - 142°C/6,65 Pa), on obtient 153 g de 1-(2'-F-octyléthylthio)3-butyloxy 2-propanol sous forme d'une huile incolore.

Rendement = 80%.

Analyse élémentaire :

| | %C | %H | %S | %F |
|---|---|---|---|---|
| Calculé | 33,45 | 3,14 | 5,25 | 52,92 |
| Mesuré | 33,52 | 3,23 | 5,14 | 52,67 |

EXEMPLE 4

**1-(2'-F-octyléthylthio)3-phénoxy 2-propanol**

A la température de 25°C, sous agitation et sous courant d'azote, on additionne à 144 g (0,3 mole) de 2-F-octyléthanethiol en solution dans 250 ml d'éther diisopropylique, 2,65 g d'une solution méthanolique de méthylate de sodium (5,65 meq $g^{-1}$).

Le mélange est chauffé à 60°C. En maintenant la température entre 55 et 65°C, on ajoute goutte à goutte, en 45 minutes, 45 g (0,3 mole) de phénylglycidyléther.

En fin de réaction, le mélange est neutralisé par 15 ml de HCl normal.

Après évaporation du solvant, on obtient un produit pâteux de couleur jaune pâle.

Le 1-(2'-F-octyléthylthio)-3-phénoxy-2-propanol est purifié par distillation (166-168°C/13,3 Pa).

On obtient 115 g d'un solide blanc.

Rendement : 65%
Point de fusion : 64°C.

Analyse élémentaire :

| | %C | %H | %S | %F |
|---|---|---|---|---|
| Calculé | 36,20 | 2,40 | 5,09 | 51,24 |
| Mesuré | 36,20 | 2,38 | 4,94 | 51,19 |

EXEMPLE 5

**1-(2'-F-hexyléthylthio)3-dodécyloxy 2-propanol**

Selon le mode opératoire décrit à l'exemple 1, on condense en 1 heure 30 minutes 121 g (0,5 mole) de dodécylglycidyléther sur 190 g (0,5 mole) de 2-F-hexyléthanethiol en présence de 4,42 g de solution méthanolique de méthylate de sodium (5,65 meq $g^{-1}$).

En fin de réaction, le mélange est neutralisé par 25 ml de HCl normal.

Le produit est purifié par distillation moléculaire en deux passages.

*1er passage:*

Température de l'huile = 80-90°C sous un vide de 0,13 Pa.
On obtient 180 g de produit brut.

*2ème passage :*

Température de l'huile = 130°C sous un vide de 0,13 Pa, le produit attendu distille.

On obtient 157 g d'une huile parfaitement incolore qui est le 1-(2'-F-hexyléthylthio)3-dodécyloxy 2-propanol.

Rendement = 50%.

Analyse élémentaire :

| | %C | %H | %S | %F |
|---|---|---|---|---|
| Calculé | 44,37 | 5,67 | 5,15 | 39,67 |
| Mesuré | 44,34 | 5,63 | 4,92 | 39,83 |

EXEMPLE 6

**1-(2'-F-hexyléthylthio)2-décanol**

Selon le mode opératoire décrit à l'exemple 1, on condense en 1 heure 30 minutes 78 g (0,5 mole) de 1,2-époxydécane avec 130 g (0,5 mole) de 2-F-hexyléthanethiol en présence de 4,4 g de solution méthanolique de méthylate de sodium (5,65 meq $g^{-1}$).

En fin de réaction, le mélange est neutralisé par 25 ml de HCl Normal.

Après distillation (165-170°C/66,5 Pa), on obtient 216 g d'un solide amorphe de couleur blanche qui est le 1-(2'-F-hexyléthylthio) 2-décanol.

Rendement = 81%.
Point de fusion = 47°C.

Analyse élémentaire :

| | %C | %H | %S | %F |
|---|---|---|---|---|
| Calculé | 40,30 | 4,70 | 5,98 | 46,04 |
| Mesuré | 40,06 | 4,62 | 6,13 | 45,63 |

EXEMPLE 7

**1-(2'-F-hexyléthylthio)2-hexanol**

Selon le mode opératoire décrit à l'exemple 1, on condense en 1 heure 30 minutes 50 g (0,5 mole) de 1,2-époxyhexane avec 190 g (0,5 mole) de 2-F-hexyléthanethiol en présence de 4,4 g de solution méthanolique de méthylate de sodium (5,65 meq $g^{-1}$).

En fin de réaction, le mélange est neutralisé par 25 ml de HCl Normal.

Après distillation (128°C/133 Pa), on obtient 171 g de 1-(2'-F-hexyléthylthio)2-hexanol sous la forme d'une huile parfaitement incolore.

Rendement = 71%.

Analyse élémentaire :

| | %C | %H | %S | %F |
|---|---|---|---|---|
| Calculé | 35,01 | 3,57 | 6,68 | 51,42 |
| Mesuré | 34,77 | 3,49 | 6,72 | 51,92 |

EXEMPLE 8

**1-(2'-F-octyléthylthio)2-hexanol**

Selon le mode opératoire décrit à l'exemple 1, on condense en 1 heure, 30 g (0,3 mole) de 1,2-epoxyhexane avec 144 g (0,3 mole) de 2-F-octyléthanethiol en présence de 2,7 g de solution méthanolique de méthylate de sodium (5,65 meq $g^{-1}$).

En fin de réaction, le mélange est neutralisé par 15 ml de HCl Normal.

Après distillation (154°C/133 Pa), on obtient 115 g d'un solide blanc amorphe qui est le 1-(2'-F-octyléthylthio)2-hexanol.

Rendement = 67%.
Point de fusion = 45°C.

Analyse élémentaire :

| | %C | %H | %S | %F |
|---|---|---|---|---|
| Calculé | 33,11 | 2,95 | 5,53 | 55,65 |
| Mesuré | 33,26 | 2,93 | 5,30 | 55,60 |

EXEMPLE 9

**1-(2'-F-hexyléthyloxy)-3-(2"-éthylhexyloxy-2-propanol**

Dans un réacteur de 1 litre, on introduit 546 g (1,5 mole) de 2-F-hexyléthanol.

On ajoute à 25°C et sous atmosphère d'azote 5,61 g de tertiobutylate de potassium. Le mélange est agité à 25°C pendant 30 minutes pour solubiliser le tertiobutylate dans le 2-F-hexyléthanol.

Le mélange est porté à 150°C et on additionne en 75 minutes 93 g (0,5 mole) de 2-éthylhexylglycidyléther.

Après 24 heures de réaction à 150°C, on ajoute 5,61 g de tertiobutylate de potassium. On renouvelle cette opération deux fois à des intervalles de temps de 24 heures.

Le 2-F-hexyléthanol en excès est alors évaporé et on obtient par distillation 68 g (20%) de 1-(2'-F-hexyléthoxy)-3-(2"-éthylhexyloxy)-2-propanol.

Point d'ébullition = 145°C sous 13,3 Pa.

Analyse élémentaire :

| | %C | %H | %F |
|---|---|---|---|
| Calculé | 41,46 | 4,94 | 44,87 |
| Mesuré | 41,55 | 4,99 | 45,01 |

EXEMPLE 10

**Mélange de 1-(2'-F-hexyléthoxy)-3-(2"-éthylhexyloxy)-2-propanol et de 2-(2'-F-hexyléthoxy)-3-(2"-éthylhexyloxy)-1-propanol (mélange 70-30)**

Dans un réacteur de 2 litres, on introduit 1640 g (4,5 moles) de 2-F-hexyléthanol. Sous atmosphère d'azote et à 25°C, on ajoute 4,4 ml d'éthérate de trifluorure de bore.

Le mélange est porté à 80°C et 111,6 g (0,6 mole) de 2'-éthylhexyl glycidyléther sont additionnés goutte à goutte en 1 heure en maintenant la température entre 79° et 82°C. A la fin de l'addition, le mélange est chauffé 90 minutes à 80°C ± 2°C. Après retour à température ambiante, le mélange est lavé deux fois par 300 ml d'eau osmosée puis par 300 ml d'eau pH=8 (NaOH). Après décantation, la phase organique est distillée. Le 2-F-hexyléthanol est éliminé dans un premier temps (40-42°C/6,65 Pa), puis le produit mixte est obtenu avec un rendement de 70% (230 g) sous la forme d'un mélange d'isomères dont la proportion relative a été déterminée par RMN $^{13}C$.
1-(2'-F-hexyléthoxy)-3-(2"-éthylhexyloxy)-2-propanol: 70%
2-(2'-F-hexyléthoxy)-3-(2"-éthylhexyloxy)-1-propanol: 30%.

Analyse élémentaire :

| | %C | %H | %F |
|---|---|---|---|
| Calculé | 41,46 | 4,94 | 44,87 |
| Mesuré | 41,64 | 4,98 | 44,71 |

EXEMPLE 11

**1-(2'-F-hexyléthylthio)-3-(2"-hexyldécyloxy)-2-propanol**

Dans un réacteur de 250 ml, placé sous courant d'azote, on introduit à 25°C 63,4 g (0,17 mole) de 2-F-hexyléthanethiol puis 0,35 g de pyridine. Le mélange est chauffé progressivement pour atteindre 70°C en 40 minutes.

En maintenant la température à 70°C, on additionne en 25 minutes 49,7 g (0,17 mole) de 2-hexyldécylglycidyléther.

Lorsque l'addition est terminée, la température est maintenue 18 heures à 70°C. Après retour à 25°C, le produit est traité pour 200 ml d'eau, pH = 5 (par addition de HCl). Après décantation, suivie de deux lavages successifs de la phase organique récupérée par chaque fois 200 ml d'eau distillée, le produit est étêté par distillation sous vide (huile 220°C - vide 1,33 Pa).

Le produit est ensuite purifié par distillation moléculaire (huile 190°C - vide 0,13 Pa). On obtient ainsi 74 g (66%) d'une huile ambrée.

Analyse élémentaire :

| | %C | %H | %S | %F |
|---|---|---|---|---|
| Calculé | 47,70 | 6,39 | 4,72 | 36,39 |
| Mesuré | 47,07 | 6,33 | 4,66 | 36,08 |

EXEMPLE 12

**1-(2'-F-octyléthyloxy)-3-(octylthio)-2-propanol**

Selon le mode opératoire décrit à l'exemple 1, on condense en 75 minutes, 130,5 g (0,25 mole) de 2-F-octyléthylglycidyléther sur 36,5 g (0,25 mole) d'octanethiol, en présence de 2,21 g de solution méthanolique de méthylate de sodium (5,65 meq g-1). En fin de réaction, le mélange est neutralisé par 12,5 ml de HCl normal.

Après distillation (172°C/266 Pa), on obtient 76 g de 1-(2'-F-octyl éthyloxy)-3-(octylthio)-2-propanol qui se présente, à pression atmosphérique, sous forme d'un solide blanc de point de fusion égal à 30°C. Les spectres RMN $^{13}C$ et de masse sont conformes à la structure attendue.

Analyse élémentaire :

| | %C | %H | %S | %F |
|---|---|---|---|---|
| Calculé | 37,84 | 4,08 | 4,81 | 48,46 |
| Mesuré | 37,95 | 4,08 | 4,72 | 48,40 |

EXEMPLE 13

**1-(2'-F-hexyléthanesulfoxy)-3-(2"-éthylhexyloxy)-2-propanol**

Dans un réacteur de 100 ml, on mélange 8,5 ml d'eau oxygénée à 30% avec 0,1 ml d'acide acétique. On ajoute à 25°C 28,3 g (0,05 mole) de 1-(2'-F-hexyléthanethio)-3-(2"-éthylhexyloxy)-2-propanol préparé à l'exemple 1. La température s'élève à 42°C. Après six heures de réaction, on ajoute 50 ml de dichlorométhane et récupère la phase organique par décantation.

Après trois lavages de la phase organique avec 20 ml d'eau distillée, la solution est filtrée puis. le solvant est évaporé sous pression réduite.

On obtient alors 23 g (80%) d'une huile visqueuse incolore dont les spectres RMN $^{13}C$ et de masse confirment la structure comme étant celle du 1-(2'-F-hexyléthanesulfoxy)-3-(2"-éthylhexyloxy)-2-propanol.

Analyse élémentaire :

| | %C | %H | %S | %F |
|---|---|---|---|---|
| Calculé | 39,20 | 4,51 | 5,51 | 42,48 |
| Mesuré | 38,87 | 4,60 | 5,42 | 41,96 |

EXEMPLE 14

**1-(2'-F-hexyléthanesulfonyl)-3-(2"-éthylhexyloxy)-2-propanol**

Dans un réacteur de 100 ml, on mélange à 25°C, 10 ml d'eau oxygénée à 30% avec 25 ml d'acide acétique et 10 gouttes d'acide sulfurique concentré.

La température du mélange est amenée à 10°C. On ajoute à cette température un mélange de 17 g (0,3 mole) de 1-(2'-F-hexyléthanethio)-3-(2"-éthylhexyloxy)-2-propanol préparé à l'exemple 1 et de 15 ml d'acide acétique.

A la fin de l'addition, après retour à 25°C, le mélange est chauffé à 85°C pendant 3 heures.

Lorsque le mélange est revenu à 25°C, la solution est versée sur de la glace sous agitation : le produit est alors extrait au dichlorométhane. La phase organique est ensuite lavée par de l'eau distillée jusqu'au retour à pH neutre.

La solution est alors séchée sur sulfate de sodium, filtrée sur papier filtre puis le solvant évaporé sous pression réduite. On obtient 16,2 g (90%) d'un solide blanc dont le point de fusion est 47°C et dont les spectres de masse et de RMN $^{13}C$ confirment la structure comme étant celle du 1-(2'-F-hexyléthanesulfonyl)-3-(2"-éthylhexyloxy)-2-propanol.

Analyse élémentaire :

| | %C | %H | %S | %F |
|---|---|---|---|---|
| Calculé | 38,13 | 4,55 | 5,36 | 41,27 |
| Mesuré | 38,26 | 4,49 | 5,24 | 41,01 |

EXEMPLE 15

$F\text{-}(CF_2)_{\bar{n}}\text{-}C_2H_4\text{-}S\text{-}CH_2\text{-}CHOH\text{-}CH_2\text{-}O\text{-}CH_2\text{-}CH(C_2H_5)\text{-}(CH_2)_3\text{-}CH_3$ ($\bar{n}$ = 12).

150 g de produit constitué d'un mélange de thiols de formule : $F\text{-}(CF_2)_n$ - $C_2H_4SH$ avec n ≥ 10 et présentant un indice de fonction SH égal à 1,50 meq $g^{-1}$ (ce qui correspond à une valeur moyenne de $\bar{n}$ = 12), produit commercialisé par la Société ATOCHEM sous la dénomination "FORALKYL EM 10N", sont fondus à 80°C dans un réacteur de 500 ml sous courant d'azote.

Sous agitation, on additionne 3 g de solution méthanolique de méthylate de sodium (solution dosée à 5,65 meq $g^{-1}$).

A la température de 85°C, on additionne 41,85 g de 2-éthylhexyl-éther de glycidyle en 40 minutes. On obtient une huile orangée qui fige à la température ambiante.

Le produit est purifié par distillation moléculaire. On obtient 150 g d'une cire blanche dont le point de fusion (Appareil "Mettler FP 89") est de 68°C.

La chromatographie en phase vapeur indique que le produit est constitué du mélange de produits :

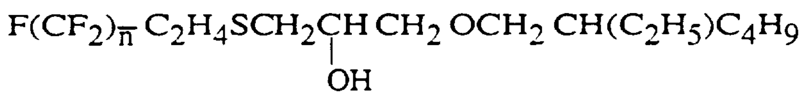

dans les proportions suivantes :

| | |
|---|---|
| n = 10 : | 10 % |
| n = 12 : | 53 % |
| n = 14 : | 27,6 % |
| n = 16 : | 8,2 % |
| n = 18 : | 1 % |
| n = 20 : | 0,2 % |

Analyse élémentaire :

| | C | H | S | F |
|---|---|---|---|---|
| Mesuré | 34,89 | 3,46 | 3,70 | 52,05 |

EXEMPLE 16

Mélange de 1-(2'-F-octyléthoxy)-hexan-2-ol et de 2-(2'-F-octyl éthyloxv)-hexan-1-ol (49/51)

Dans un réacteur de 2 litres, on introduit 1392 g (3 moles) de 2-F-octyléthanol. Sous $N_2$ et sous agitation, on ajoute 5 ml de $BF_3/Et_2O$ à 25°C. Le mélange est porté à 80°C et 60 g (0,6 mole) d'époxy-1,2-hexane sont ajoutés goutte à goutte en maintenant la température entre 80 et 85°C. A la fin de l'addition, le mélange est maintenu sous agitation à 80°C pendant 1 heure.

Après retour à 25°C, le mélange est lavé deux fois par 300 ml d'eau osmosée, une fois par 300 ml d'eau (pH 11 - NaOH) puis une dernière fois par 300 ml d'eau osmosée.

Après décantation, le 2-F-octyléthanol en excès est évaporé sous pression réduite (70°C/60 Pa). Le mélange d'isomères 1-(2'-F-octyléthoxy)-hexan-2-ol et 2-(2'-F-octyléthyloxy)-hexan-1-ol en proportion 49/51 - dosage effectué par RMN $^{13}C$ quantitative - est distillé à 110°C sous 40 Pa.

On obtient 236 g (70%) d'une huile visqueuse incolore.

## EXEMPLES DE FORMULATION

### EXEMPLE 1

On prépare un shampooing en mélangeant les composés suivants :

| | |
|---|---|
| - Composé de l'exemple 1 | 0,2 g |
| - Lauryléthersulfate de sodium $C_{12}$-$C_{14}$ (70/30) à 2,2 moles d'oxyde d'éthylène, vendu en solution aqueuse à 28% sous la dénomination EMPICOL ESB/3FL par la Société MARCHON | 10 g MA |
| - Mélange cocoylamidopropylbétaïne/monolaurate de glycérol, vendu en solution aqueuse à 35% sous la dénomination TEGO BETAINE HS par la Société GOLDSCHMIDT | 5 g MA |
| - Chlorure de sodium | 1 g |
| - Hydroxyéthylcellulose vendue sous la dénomination NATROSOL 250 HHR par la Société AQUALON | 0,5 g |
| - Acide chlorhydrique qs pH=5,5 | |
| - Conservateurs, parfum qs | |
| - Eau déminéralisée qsp | 100 g |

Les cheveux lavés avec ce shampooing sont brillants et faciles à démêler.

On obtient le même résultat en remplaçant, dans la formulation pour shampooing ci-dessus, le composé de l'exemple de préparation 1 par celui de l'exemple de préparation 10.

### EXEMPLE 2

On prépare un après-shampooing en mélangeant les composés suivants :

| | |
|---|---|
| - Composé de l'exemple 2 | 0,5 g |
| - Polymère carboxylique vendu sous la dénomination CARBOPOL 940 par la Société GOODRICH | 0,5 g |
| - Triéthanolamine qs pH=7 | |
| - Eau déminéralisée qsp | 100 g |

Les cheveux traités avec cet après-shampooing sont lisses et brillants.

### EXEMPLE 3

On prépare une pâte dentifrice en mélangeant les composés suivants :

| | |
|---|---|
| - Composé de l'exemple 2 | 0,5 g |
| - Silice précipitée amorphe, vendue sous la dénomination TIXOSIL 333 par la Société RHONE POULENC | 8 g |
| - Silice précipitée amorphe, vendue sous la dénomination TIXOSIL 73 par la Société RHONE POULENC | 12 g |
| - Sel de sodium de carboxyméthylcellulose, vendu sous la dénomination BLANOSE 9M31F par la Société AQUALON | 1,3 g |
| - Laurylsulfate de sodium vendu sous la dénomination EMPICOL LXV/E par la Société MARCHON | 1,8 g |
| - Sorbitol à 70 % | 30 g |
| - Dioxyde de titane | 1 g |
| - Parahydroxybenzoate de méthyle | 0,2 g |
| - Fluorure de sodium | 0,22 g |
| - Arôme qs | |
| - Saccharinate de sodium | 0,15 g |
| - Eau déminéralisée qsp | 100 g |

EXEMPLE 4

On prépare un stick antitranspirant en mélangeant les composés suivants :

| | |
|---|---|
| - Composé de l'exemple 1 | 1,0 g |
| - Alcool stéarylique | 25 g |
| - Hydroxychlorure d'aluminium anhydre micro moulu | 17,5 g |
| - Talc vendu par la Société LUZENAC | 2 g |
| - Butylhydroxytoluène | 0,025 g |
| - Parfum qs | |
| - Mélange cyclopentadiméthylsiloxane/cyclotétradiméthylsiloxane/cyclohexadiméthylsiloxane (62 à 72/4/24), vendu sous la dénomination DC 345 FLUID par la Société DOW CORNING qsp | 100 g |

EXEMPLE 5

On prépare une huile moussante pour le bain en mélangeant les composés suivants :

| | |
|---|---|
| - Composé de l'exemple 2 | 2 g |
| - Mélange 50/50 de lauryléthersulfate de monoisopropanolamine/diéthanolamide d'acide de coprah, vendu sous la dénomination TEXAPON WW99 par la Société HENKEL | 40 g |
| - Monolaurate de sorbitan à 20 moles d'oxyde d'éthylène, vendu sous la dénomination TWEEN 20 par la Société ICI | 5 g |
| - Huile de sésame vierge | 25 g |
| - Butylhydroxytoluène | 0,1 g |
| - Butylhydroxyanisole | 0,04 g |
| - Parahydroxybenzoate de propyle | 0,15 g |
| - Parfum qs | |
| - Huile de colza raffinée désodorisée qsp | 100 g |

EXEMPLE 6

On prépare un bâton de rouge à lèvres ayant la composition suivante :

| | |
|---|---|
| - Ozokerite | 14,9 g |
| - Cire microcristalline | 4,9 g |
| - Cire de Candellila | 7,4 g |
| - Composé de l'exemple 1 | 1,0 g |
| - Huile de Jojoba | 6,2 g |
| - Huile de ricin | 1,2 g |
| - Lanoline | 18,6 g |
| - Lanoline acétylée | 9,9 g |
| - Huile de vaseline | 11,1 g |
| - Talc | 3,7 g |
| - Mica-titane | 8,7 g |
| - D and C Red 7 calcium lake | 5,2 g |
| - D anc C Red 7 Barium lake | 2,8 g |
| - FDC Yellow 5 | 1,0 g |
| - Dioxyde de titane | 3,1 g |
| - Butylhydroxytoluène | 0,3 g |
| - Parfum qs | |

en mélangeant les huiles à une température de 50 à 60°C. Les pigments et laques organiques sont broyés dans la phase huileuse.

On ajoute alors les cires fondues, le talc et le micatitane puis le parfum.

La composition est alors coulée dans un moule.

L'application du rouge à lèvres est aisée (glisse facilement) et celui-ci confère de la douceur aux lèvres.

EXEMPLE 7

On prépare une émulsion solaire H/E de composition suivante :

| | |
|---|---|
| - Mélange d'alcool cétylstéarylique et d'alcool cétylstéarylique oxyéthyléné à 33 moles d'oxyde d'éthylène, vendu sous la dénomination SINNOWAX AO par la Société HENKE2 | 7 g |
| - Mélange de mono- et distéarate de glycérol non auto-émulsionnable | 2 g |
| - Alcool cétylique | 1,5 g |

Suite du Tableau sur la page suivante

(suite)

| | |
|---|---|
| - Huile de silicone | 1,5 g |
| - Composé de l'exemple 1 | 10 g |
| - Paraméthoxycinnamate de 2- éthylhexyle, vendu sous la dénomination PARSOL MCX par la Société GIVAUDAN | 5 g |
| - Glycérine | 15 g |
| - Conservateurs qs | |
| - Eau purifiée qsp | 100 g |

en dissolvant d'abord le filtre dans la phase grasse contenant les émulsionnants, puis en chauffant cette phase grasse à une température de 80 à 85°C et en ajoutant, sous vive agitation, l'eau préalablement chauffée à une température d'environ 80°C.

La composition, d'application aisée sans effet collant, confère de la douceur à la peau tout en la protégeant du rayonnement UV.

EXEMPLE 8

On prépare une émulsion H/E ayant la composition suivante :

| | |
|---|---|
| - Triglycérides d'acides caprique/caprylique, vendus sous la dénomination MIGLYOL 812 par la Société DYNAMIT NOBEL | 11 g |
| - Composé de l'exemple 1 | 5 g |
| - Glycérol | 2 g |
| - Triéthanolamine | 0,80 g |
| - Conservateurs qs | |
| - Acide polyacrylique réticulé modifié, vendu sous la dénomination PEMULEN TR 2 par la Société GOODRICH | 0,1 g |
| - Acide polyacrylique réticulé, vendu sous la dénomination CARBOPOL 940 par la Société GOODRICH | 0,6 g |
| - Eau qsp | 100 g |

en mélangeant; à froid, dans la phase aqueuse, les acides polyacryliques réticulés d'une part et, d'autre part, en mélangeant les autres ingrédients qui constituent la phase grasse que l'on introduit ensuite dans la phase aqueuse par agitation.

EXEMPLE 9

On réalise une émulsion H/E de la même façon qu'à l'exemple 8, en remplaçant le composé de l'exemple 1 par le composé de l'exemple 2.

Les compositions des exemples 8 à 9, d'application aisée sans effet collant, confèrent de la douceur à la peau.

EXEMPLE 10

On prépare une laque capillaire aérosol ayant la composition suivante :

| | |
|---|---|
| - Composé de l'exemple 2 | 0,4 g |
| - Copolymère acétate de vinyl-90/acide crotonique-10 neutralisé à 100% par l'aminométhylpropanol | 3,2 g |
| - Ethanol | 33,4 g |
| - Diméthyléther | 43,0 g |
| - Pentane | 20,0 g |

La chevelure laquée avec cette composition présente une brillance et une facilité de brossage améliorée.

EXEMPLE 11

On prépare un vernis à ongles ayant la composition suivante :

| | |
|---|---|
| - Nitrocellulose | 10,82 g |
| - Résine toluène sulfonamide formaldéhyde, vendue sous la dénomination KETJENFLEX MS 80 par la Société AKZO | 9,74 g |
| - Acétylcitrate de tributyle vendu sous la dénomination CITROFLEX A4 par la Société PFIZER | 6,495 g |
| - Toluène | 30,91 g |
| - Acétate de butyle | 21,64 g |
| - Acétate d'éthyle | 9,27 g |
| - Alcool isopropylique | 7,72 g |
| - Stéaralkonium hectorite | 1,35 g |
| - Pigments | 1,00 g |
| - Acide citrique | 0,055 g |
| - Composé de l'exemple 1 | 1,00 g |

Le composé de l'exemple 1 peut être remplacé par les composés des exemples de préparation 8, 9 ou 16.

L'étalement du vernis sur l'ongle est aisé et on obtient un film présentant une très bonne adhésion sur l'ongle ainsi qu'une bonne brillance. On observe également une bonne tenue de la brillance dans le temps et une bonne résistance du film à l'abrasion.

EXEMPLE 12

On prépare un shampooing de composition suivante :

| | |
|---|---|
| - Alkyl($C_9$-$C_{10}$-$C_{11}$/20-40-40)polyglycoside (1,4), vendu à 50% de MA par la Société HENKEL sous la dénomination APG 300 | 15 g MA |
| - Composé de l'exemple 1 | 1 g |
| - Parfum, conservateur qs | |
| - HCl qs pH=7 | |
| - Eau qsp | 100 g |

Les cheveux lavés avec ce shampooing sont brillants et faciles à démêler.

EXEMPLE 13

On prépare un shampooing de composition suivante :

| | |
|---|---|
| - Tensio-actif non-ionique de type hydroxypropyléther obtenu par condensation, en catalyse alcaline, de 3,5 moles de glycidol sur un mélange d'alphadiols en $C_{11}$-$C_{14}$ selon le brevet français n° 2.091.516 | 10 g |
| - Composé de l'exemple 1 | 0,5 g |
| - Parfum, conservateur qs | |
| - pH spontané = 5,8 | |
| - Eau qsp | 100 g |

Les cheveux lavés avec ce shampooing sont brillants et faciles à démêler.

EXEMPLE 14

**STICK POUR LE SOIN DES LEVRES**

On mélange à chaud (50-60°C) les ingrédients suivants :

| | |
|---|---|
| - Cire de Carnauba | 10 g |
| - Cire microcristalline | 6 g |
| - Huile de ricin | 30 g |
| - Lanoline | 25 g |
| - Composé de l'exemple 1 | 2 g |
| - Huile de vaseline | 12 g |
| - Lanolate d'isopropyle | 14 g |
| - Acétate de tocophérol | 0,5 g |
| - Palmitate de vitamine A | 0,5 g |

On coule le mélange dans un moule.

Après refroidissement, on obtient un stick de soin des lèvres qui est facile à appliquer - il glisse facilement - et qui confère de la douceur aux lèvres.

EXEMPLE 15

**FOND DE TEINT**

On prépare un fond de teint à partir des constituants suivants :

| | |
|---|---|
| - Eau déminéralisée | 53,01 g |
| - Propylèneglycol | 6,5 g |
| - Silicate de magnésium et d'aluminium | 1,12 g |
| - Oxyde de fer jaune | 0,8 g |
| - Oxyde de fer brun | 0,67 g |
| - Oxyde de fer noir | 0,23 g |
| - Oxyde de titane | 5,3 g |
| - Parahydroxybenzoate de propyle | 0,05 g |
| - Parahydroxybenzoate d'éthyle | 0,20 g |
| - Triéthanolamine | 0,93 g |
| - Mélange de mono- et distéarate non autoémulsionnable | 0,37 g |
| - Composé de l'exemple 1 | 5 g |
| - Cyclopentadiméthylsiloxane | 14 g |
| - Acide stéarique | 1,86 g |
| - Stéarate de polyéthylèneglycol à 2 moles d'oxyde d'éthylène | 0,56 g |
| - Trichloro-2,4,4'-hydroxy 2'-diphényléther, vendu sous la dénomination "IRGASAN DP 300" par la Société CIBA GEIGY | 0,1 g |
| - Poudre de polyéthylène | 9,3 g |

On chauffe l'eau à 90°C, on y disperse le propylèneglycol et on agite. On refroidit à 70°C, on ajoute le silicate, les pigments et les conservateurs. On agite jusqu'à homogénéité. On stabilise la température à 60°C, puis on ajoute la triéthanolamine. On stabilise la température à 60°C et on introduit la phase grasse préalablement préparée contenant l'IRGASAN DP 300. On réalise une émulsion huile-dans-eau par agitation. A 40°C, on ajoute une poudre de polyéthylène et on refroidit.

EXEMPLE 16

**SERUM SOIN DES ONGLES**

On réalise le mélange suivant :

| | |
|---|---|
| - Cyclopentadiméthylsiloxane | 99,4 g |

Suite du Tableau sur la page suivante

(suite)

| | |
|---|---|
| - Acétate de tocophérol | 0,1 g |
| - Composé de l'exemple 1 | 0,5 g |

Ce produit s'applique sur l'ongle en massage. Après un temps suffisant pour permettre à la silicone de s'évaporer, on peut appliquer un vernis sur l'ongle sans que sa tenue ne soit altérée.

EXEMPLE 17

**CREME**

**Phase A**

| | | |
|---|---|---|
| - Alcool cétylique | 4 | g |
| - Tristéarate de sorbitan | 0,9 | g |
| - Stéarate de polyéthylèneglycol à 40 moles d'oxyde d'éthylène | 2 | g |
| - Stéarate de glycéryle | 3 | g |
| - Myristate de myristyle | 2 | g |
| - Palmitate d'octyle | 5 | g |
| - Polyisobutène hydrogéné vendu sous la dénomination "PARLEAM" par la Société NIPPON OIL & FATS | 6,5 | g |

**Phase B**

| | | |
|---|---|---|
| - Perfluoropolyéther vendu sous la dénomination "FOMBLIN HCR" par la Société AUSIMONT | 10,4 | g |
| - Composé de l'exemple 14 | 5 | g |

**Phase C**

| | | |
|---|---|---|
| - Conservateurs | 0,35 | g |
| - Glycérol | 8 | g |
| - Eau | 16,25 | g |

**Phase D**

| | | |
|---|---|---|
| - Eau | 35,3 | g |
| - Imidazolidinylurée | 0,3 | g |

On chauffe la phase A à 80°C, on y ajoute la phase B, on maintient à 80°C. On chauffe la phase C à 80°C et on

l'ajoute dans les phases A et B. On émulsionne à 80°C, puis on abaisse la température à 45°C. On ajoute la phase C sous vive agitation.

EXEMPLE 18

**CREME**

Selon le même mode opératoire qu'à l'exemple 17, on prépare une crème de composition suivante :

| | |
|---|---|
| **Phase A** | |
| - Alcool cétylique | 4 g |
| - Tristéarate de sorbitan | 0,9 g |
| - Stéarate de polyéthylèneglycol à 40 moles d'oxyde d'éthylène | 2 g |
| - Stéarate de glycéryle | 3 g |
| - Myristate de myristyle | 2 g |
| - Palmitate d'octyle | 5 g |
| - Polyisobutène hydrogéné | 6,5 g |
| **Phase B** | |
| - Composé de l'exemple 14 | 3 g |
| **Phase C** | |
| - Conservateurs | 0,35 g |
| - Glycérol | 30 g |
| - Eau | 17 g |
| **Phase D** | |
| - Imidazolidinylurée | 0,35 g |
| - Eau | 25,9 g |

EXEMPLE 19

On prépare, selon le même mode opératoire qu'à l'exemple 6, un bâton de rouge à lèvres ayant la composition suivante :

| | |
|---|---|
| - Ozokérite | 15 g |
| - Cire d'abeille | 8 g |
| - Cire de Carnauba | 4 g |
| - Composé de l'exemple 12 | 2 g |
| - Huile de jojoba | 7 g |
| - Huile de sésame | 10 g |
| - Huile de ricin | 2 g |
| - Lanoline | 20 g |
| - Lanoline acétylée | 10 g |
| - Huile de vaseline | 10 g |
| - Oxyde de titane | 3 g |
| - FD & C Yellox n°6 aluminium lake | 3 g |
| - DC Red n°7 calcium lake | 6 g |
| - Parfum qs | |

L'application du rouge à lèvres est aisée (glisse facilement) et celui-ci confère de la douceur aux lèvres.

**Revendications**

1. Composés de formule :

$$R_F - (CH_2)_n - X - [C_3H_5(OH)] - (Y)_x - R_H \qquad (I)$$

dans laquelle $C_3H_5(OH)$ représente les structures :

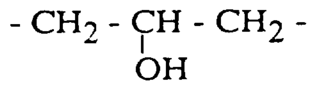 ou 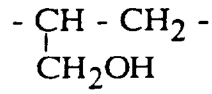

(Ia) (Ib)

$R_F$ représente un radical alkyle perfluoré en $C_4$-$C_{20}$ ou un mélange de radicaux alkyle perfluorés en $C_4$-$C_{20}$;
$R_H$ représente un radical alkyle linéaire ou ramifié en $C_1$-$C_{22}$ ou un mélange de radicaux alkyle linéaires ou ramifiés en $C_1$-$C_{22}$ ou un radical aryle ou aralkyle;
n est compris entre 0 et 4;
X représente O, S ,

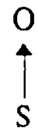 ou 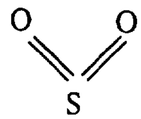 ;

x représente O ou 1 ;
Y représente O, S,

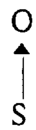 ou 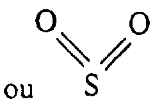 ;

sous réserve que lorsque X = S,

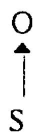 ou 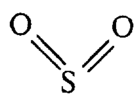 ,

Y n'est pas S,

, 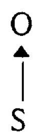 ou 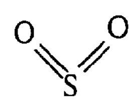 .

2. Composés selon la revendication 1, caractérisés en ce que :

$R_F$ représente un radical alkyle perfluoré en $C_6$-$C_{12}$;
$R_H$ représente un radical alkyle linéaire ou ramifié en $C_3$-$C_{18}$, un radical aryle en $C_6$-$C_{10}$ ou un radical aralkyle en $C_7$-$C_{15}$,
n est 2,
X représente O ou S, et
Y représente O.

3. Utilisation des composés de formule (I) selon la revendication 1 ou 2, dans des compositions cosmétiques.

4. Composition cosmétique comportant au moins un composé selon la revendication 1 ou 2.

5. Composition cosmétique selon la revendication 4, caractérisée en ce qu'elle est sous forme de lait, crème, lotion huileuse ou oléo-alcoolique, gel gras ou oléoalcoolique, dispersion vésiculaire à base de lipides amphiphiles ioniques ou non-ioniques, bâtonnet solide, pâte, spray ou mousse aérosol.

**6.** Composition cosmétique selon la revendication 4 ou 5, caractérisée en ce qu'elle est sous forme de lait, de crème de soin pour la peau ou les cheveux, de crème, lotion ou lait démaquillant, de crème, gel, lotion ou lait anti-solaire, de crème ou mousse de rasage, de lotion après-rasage, de shampooing ou d'après-shampooing, de déodorant corporel, de pâte dentifrice, de laque, de produit de maquillage des cils, sourcils, ongles, lèvres ou peau, de crème de traitement de l'épiderme, de fond de teint, de bâton de rouge à lèvres, de fard à paupières ou à joues, d'eye-liner, mascara ou de produit de soin pour les lèvres ou les ongles.

**7.** Composition cosmétique selon la revendication 4 ou 5, caractérisée en ce qu'elle comporte 0,1 à 25% en poids du composé de formule (I).

**8.** Composition cosmétique selon l'une des revendications 4 à 7, caractérisée en ce qu'elle comporte un ou plusieurs adjuvants choisis dans le groupe formé des corps gras usuels, des solvants organiques, des silicones, les épaississants, les adoucissants, des filtres solaires UV-A ou UV-B ou à bande large, des agents anti-mousses, des agents hydratants, des humectants, des parfums, des conservateurs, des tensio-actifs, des charges, des séquestrants, des émulsionnants, des polymères anioniques, cationiques, non-ioniques, amphotères ou leurs mélanges, des anti-perspirants, des agents alcalinisants, des colorants, des pigments, des agents propulseurs, des anti-oxydants et des anti-radicaux libres.

**9.** Procédé de préparation des composés de formule (I) :

$$R_F-(CH_2)_n-X-[C_3H_5(OH)]-(Y)_x-R_H \quad (I)$$

dans laquelle $C_3H_5(OH)$ représente les structures :

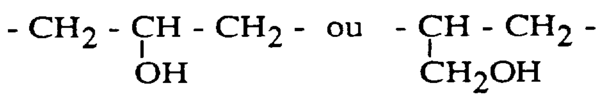

ou

(Ia) (Ib)

$R_F$ représente un radical alkyle perfluoré en $C_4$-$C_{20}$ ou un mélange de radicaux alkyle perfluorés en $C_4$-$C_{20}$;
$R_H$ représente un radical alkyle linéaire ou ramifié en $C_1$-$C_{22}$ ou un mélange de radicaux alkyle linéaires ou ramifiés en $C_1$-$C_{22}$ ou un radical aryle ou aralkyle;
n est compris entre 0 et 4;
X représente O, S ,

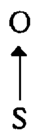 ou 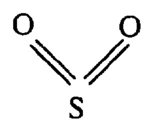 ;

x représente O ou 1;
Y représente O, S ,

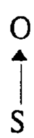 ou 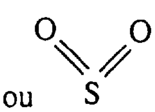 ;

sous réserve que lorsque X = S,

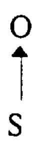 ou 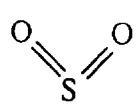 ,

Y n'est pas S,

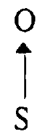 ou 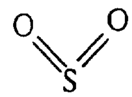 .

caractérisé en ce qu'il comporte au moins
la réaction d'un composé fluoré à hydrogène acide de formule (II) :

$$R_F\text{-}(CH_2)_n\text{-}X\text{-}H \qquad (II)$$

avec un époxyde de formule (III) :

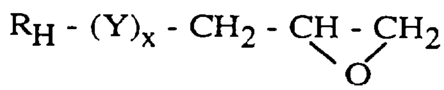 (III)

ou la réaction d'un composé hydrocarboné à hydrogène acide de formule (IV) :

$$R_H\text{-}(Y)_x\text{-}H \qquad (IV)$$

avec un époxyde fluoré de formule (V) :

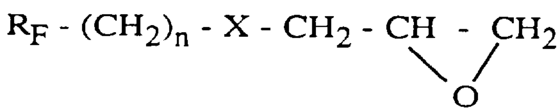 (V)

dans lesquelles $R_F$, $R_H$, n et x ont les significations ci-dessus,
X désigne O ou S, Y désigne O ou S, sous réserve que lorsque X est S, Y n'est pas S,
en présence d'un composé basique ou acide jouant le rôle de réactif ou de catalyseur; l'oxydation éventuelle de la fonction mercaptan en sulfoxyde ou sulfone avec de l'eau oxygénée et la récupération du composé de formule (I) obtenu.

**10.** Procédé selon la revendication 9, caractérisé en ce que la réaction du composé fluoré à hydrogène acide et de l'époxyde a lieu en présence ou en l'absence de solvant.

**11.** Procédé selon la revendication 9 ou 10, caractérisé en ce que ledit composé jouant le rôle de réactif ou de catalyseur est présent en une quantité comprise entre 1 et 100% molaire par rapport au composé fluoré à hydrogène acide de formule (II) ou (IV).

**12.** Procédé selon la revendication 11, caractérisé en ce que la quantité dudit composé jouant le rôle de réactif ou de catalyseur est comprise entre environ 2 et 10% molaire par rapport au composé fluoré à hydrogène acide de formule (II) ou (IV).

**Patentansprüche**

**1.** Verbindungen der Formel:

$$R_F\text{-}(CH_2)_n\text{-}X\text{-}[C_3H_5(OH)]\text{-}(Y)_x\text{-}R_H \qquad (I)$$

worin $C_3H_5(OH)$ die Strukturen darstellt:

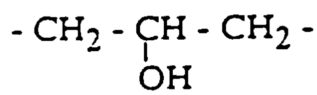 oder 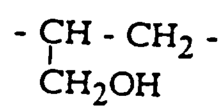

(Ia) (Ib)

und worin $R_F$ einen perfluorierten $C_{4\text{-}20}$-Alkylrest oder eine Mischung perfluorierter $C_{4\text{-}20}$-Alkylreste und
$R_H$ einen linearen oder verzweigten $C_{1-22}$-Alkylrest oder eine Mischung linearer oder verzweigter $C_{1-22}$-Alkylreste oder einen Aryl- oder Aralkylrest darstellen,
und worin ferner gilt:

n beträgt 0 bis 4;
X stellt O, S,

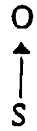 oder 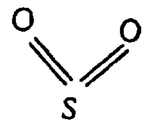

dar;
x stellt O oder 1 dar;
Y stellt O, S,

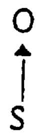 oder 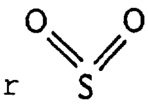

dar; mit der Maßgabe, daß, wenn X = S,

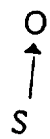 oder 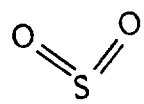 ,

Y nicht S,

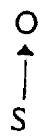 oder 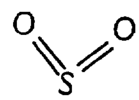

ist.

**2.** Verbindungen gemäß Anspruch 1,

dadurch **gekennzeichnet**, daß gilt:
$R_F$ stellt einen perfluorierten $C_{6-12}$-Alkylrest dar;
$R_H$ stellt einen linearen oder verzweigten $C_{3-18}$-Alkylrest, einen $C_{6-10}$-Arylrest oder einen $C_{7-15}$-Aralkyrest dar;
n ist 2;
X stellt O oder S und
Y stellt O dar.

**3.** Verwendung von Verbindungen der Formel (I) gemäß Anspruch 1 oder 2 in kosmetischen Zusammensetzungen.

**4.** Kosmetische Zusammensetzung, enthaltend mindestens eine Verbindung gemäß Anspruch 1 oder 2.

**5.** Kosmetische Zusammensetzung gemäß Anspruch 4,

dadurch **gekennzeichnet**, daß sie in Form einer Milch, Creme, ölartigen oder oleoalkoholischen Lotion, eines fettartigen oder oleoalkoholischen Gels, einer bläschenartigen Dispersion auf Basis amphiphiler ionischer oder nicht-ionischer Lipide, eines Feststoffstäbchens, einer Paste, eines Spray oder Aerosol-Schaums vorliegt.

**6.** Kosmetische Zusammensetzung gemäß Anspruch 4 oder 5,

dadurch **gekennzeichnet,** daß sie in Form einer Milch, einer Pflegecreme für die Haut oder die Haare, einer Creme, Lotion oder Milch zum Entschminken, einer Creme, eines Gels, einer Lotion oder einer Milch als Sonnenschutzmittel, einer Creme oder eines Schaums zur Rasur, einer Lotion nach einer Rasur, eines Shampoo oder Nach-Shampoo, eines Deodorant für den Körper, einer Zahnpaste, eines Lacks, eines Schminkprodukts

für die Wimpern, Augenbrauen, Nägel, Lippen oder die Haut, einer Creme zur Behandlung der Haut, einer Teint-Grundlage, eines Lippenstifts, von Lidschatten oder einer Wangenschminke, eines Liniermittels für die Augen, einer Wimperntusche oder in Form eines Pflegeprodukts für die Lippen oder die Nägel vorliegt.

**7.** Kosmetische Zusammensetzung gemäß Anspruch 4 oder 5,

dadurch **gekennzeichnet**, daß sie 0,1 bis 25 Gew.% der Verbindung der Formel (I) enthält.

**8.** Kosmetische Zusammensetzung gemäß einem der Ansprüche 4 bis 7,

dadurch **gekennzeichnet**, daß sie einen oder mehrere Hilfsstoffe enthält, ausgewählt aus der Gruppe aus üblichen Fettkörpern, organischen Lösungsmitteln, Silikonen, Verdickungsmitteln, Weichmachern, Sonnenfilterstoffen für UV-A oder UV-B oder für den langen Wellenlängenbereich, aus Antischaummitteln, hydratisierenden Mitteln, Feuchtigkeitsmitteln, Parfümstoffen, Konservierungsstoffen, oberflächenaktiven Mitteln, Beladungsmitteln, Sequestriermitteln, Emulgatoren, aus anionischen, kationischen, nicht-ionischen oder amphoteren Polymeren oder aus deren Mischungen, aus Antischweißmitteln, alkalisch stellenden Mitteln, Färbemitteln, Pigmenten, Treibmitteln, Antioxidantien und aus Abfangmitteln für freie Radikale.

**9.** Verfahren zur Herstellung von Verbindungen der Formel (I) :

$$R_F\text{-}(CH_2)_n\text{-}X\text{-}[C_3H_5(OH)]\text{-}(Y)_x\text{ - }R_H \qquad \text{(I)}$$

worin $C_3H_5(OH)$ die Strukturen darstellt:

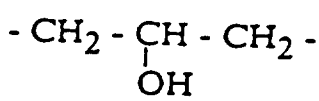

(Ia)

oder

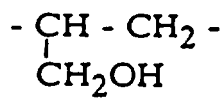

(Ib)

und worin $R_F$ einen perfluorierten $C_{4-20}$-Alkylrest oder eine Mischung perfluorierter $C_{4-20}$-Alkylreste und $R_H$ einen linearen oder verzweigten $C_{1-22}$-Alkylrest oder eine Mischung linearer oder verzweigter $C_{1-22}$-Alkylreste oder einen Aryl- oder Aralkylrest darstellen,
und worin ferner gilt:
n beträgt 0 bis 4;
X stellt O, S,

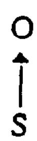

oder

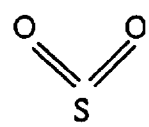

dar ;
x stellt O oder 1 dar;
Y stellt O, S,

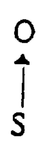

oder

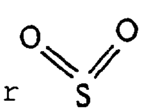

dar; mit der Maßgabe, daß, wenn X = S,

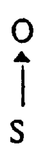

oder

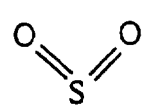

,

Y nicht S,

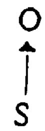

oder

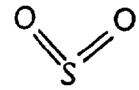

ist,

dadurch **gekennzeichnet**, daß man zumindest eine fluorierte, ein saures Wasserstoffatom aufweisende Verbindung der Formel (II):

$$R_F\text{-}(CH_2)_n\text{-}X\text{-}H \qquad (II)$$

mit einem Epoxid der Formel (III):

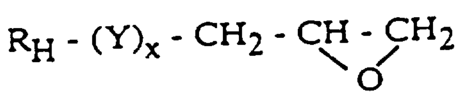

(III)

oder eine ein saures Wasserstoffatom aufweisende Kohlenwasserstoffverbindung der Formel (IV):

$$R_H\text{-}(Y)_x\text{-}H \qquad (IV)$$

mit einem fluorierten Epoxid der Formel (V):

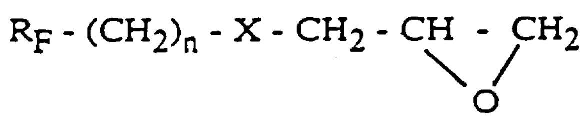

(V)

in denen $R_F$, $R_H$, n und x die obigen Bedeutungen haben, X O oder S und Y O oder S bedeuten, mit der Maßgabe, daß, wenn X S ist, Y nicht S ist,
in Gegenwart einer basischen oder sauren Verbindung zur Reaktion bringt, welche die Rolle einer reaktiven Komponente oder eines Katalysators spielen, wobei man gegebenenfalls die Mercaptan-Funktion zu einem Sulfoxid oder Sulfon mit sauerstoffhaltigem Wasser oxidiert, und daß man die erhaltene Verbindung der Formel (I) gewinnt.

**10.** Verfahren gemäß Anspruch 9,

dadurch **gekennzeichnet**, daß die Reaktion der fluorierten, ein saures Wasserstoffatom aufweisenden Verbindung und des Epoxids in Gegenwart oder Abwesenheit eines Lösungsmittels stattfindet.

**11.** Verfahren gemäß Anspruch 9 oder 10,

dadurch **gekennzeichnet**, daß die genannte Verbindung, die die Rolle einer reaktiven Komponente oder eines Katalysators spielt, in einer Menge von 1 bis 100 Mol% vorhanden ist, bezogen auf die fluorierte, ein saures Wasserstoffatom aufweisende Verbindung der Formel (II) oder (IV).

**12.** Verfahren gemäß Anspruch 11,

dadurch **gekennzeichnet**, daß die Menge der genannten Verbindung, die die Rolle einer reaktiven Komponente oder eines Katalysators spielt, ca. 2 bis 10 Mol% beträgt, bezogen auf die fluorierte, ein saures Wasserstoffatom aufweisende Verbindung der Formel (II) oder (IV).

**Claims**

**1.** Compounds of the formula:

$$R_F\text{-}(CH_2)_n - X - [C_3H_5(OH)] - (Y)_x - R_H \qquad (I)$$

in which $C_3H_5(OH)$ represents the structures:

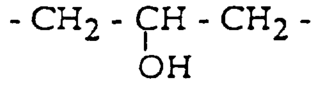

(Ia)

or

$R_F$ represents a perfluorinated $C_4$-$C_{20}$ alkyl radical or a mixture of perfluorinated $C_4$-$C_{20}$ alkyl radicals;
$R_H$ represents a linear or branched $C_1$-$C_{22}$ alkyl radical or a mixture of linear or branched $C_1$-$C_{22}$ alkyl radicals or an aryl or aralkyl radical;
n is between 0 and 4;
X represents or

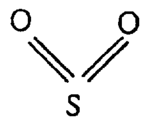 ;

x represents 0 or 1;
Y represents O, S ,

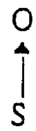 or 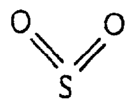 ;

with the proviso that, when X = S,

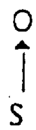 or 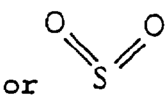 ,

Y is not S,

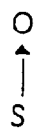 or 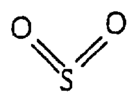 .

2. Compounds according to claim 1, characterized in that:

$R_F$ represents a perfluorinated $C_6$-$C_{12}$ alkyl radical;
$R_H$ represents a linear or branched $C_3$-$C_{18}$ alkyl radical, a $C_6$-$C_{10}$ aryl radical or a $C_7$-$C_{15}$ aralkyl radical,
n is 2,
X represents O or S, and
Y represents O.

3. Use of the compounds of formula (I) according to claim 1 or 2, in cosmetic compositions.

4. Cosmetic composition containing at least one compound according to claim 1 or 2.

5. Cosmetic composition according to claim 4, characterized in that it is in the form of a milk, cream, oily or oleoalcoholic lotion, fatty or oleoalcoholic gel, vesicular dispersion based on ionic or nonionic amphiphilic lipids, solid stick, paste, spray or aerosol foam.

6. Cosmetic composition according to claim 4 or 5, characterized in that it is in the form of a milk or cream for skin or

hair care, make-up removal cream, lotion or milk, antisun cream, gel, lotion or milk, shaving cream or foam, aftershave lotion, shampoo or after-shampoo product, body deodorant, toothpaste, lacquer, make-up product for the eyelashes, eyebrows, nails, lips or skin, cream for treating the epidermis, make-up foundation, lipstick, eyeshadow or blusher, eyeliner, mascara or lip or nail care product.

**7.** Cosmetic composition according to claim 4 or 5, characterized in that it contains 0.1 to 25% by weight of the compound of formula (I).

**8.** Cosmetic composition according to one of claims 4 to 7, characterized in that it contains one or more adjuvants chosen from the group composed of fats of a conventional nature, organic solvents, silicones, thickeners, demulcents, UV-A or UV-B or broad-band sunscreen agents, antifoams, hydrating agents, humectants, fragrances, preservatives, surfactants, fillers, sequestering agents, emulsifiers, anionic, cationic, nonionic or amphoteric polymers or mixtures thereof, antiperspirants, alkalinizing agents, colorants, pigments, propellants, antioxidants and anti-free-radical agents.

**9.** Process for preparing the compounds of formula (I):

$$R_F\text{-}(CH_2)_n - X - [C_3H_5(OH)] - (Y)_x - R_H \quad (I)$$

in which $C_3H_5(OH)$ represents the structures:

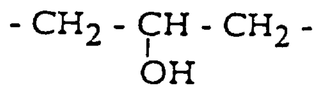
or
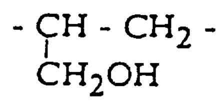

(Ia) (Ib)

$R_F$ represents a perfluorinated $C_4$-$C_{20}$ alkyl radical or a mixture of perfluorinated $C_4$-$C_{20}$ alkyl radicals;

$R_H$ represents a linear or branched $C_1$-$C_{22}$ alkyl radical or a mixture of linear or branched $C_1$-$C_{22}$ alkyl radicals or an aryl or aralkyl radical;

n is between 0 and 4;

X represents O, S,

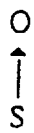
or
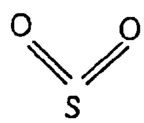
;

x represents 0 or 1;

Y represents O, S,

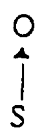
or
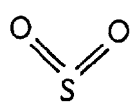
;

with the proviso that, when X = S,

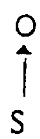
or
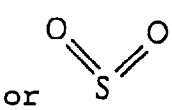
,

Y is not S,

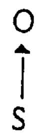 or 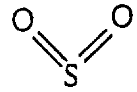 .

characterized in that it entails at least
the reaction of a fluorinated compound containing acidic hydrogen, of formula (II):

$$R_F - (CH_2)_n - X - H \quad (II)$$

with an epoxide of formula (III):

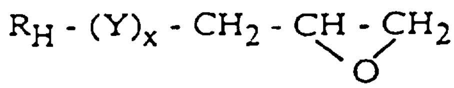 (III)

or the reaction of a hydrocarbon compound containing acidic hydrogen, of formula (IV):

$$R_H - (Y)_x - H \quad (IV)$$

with a fluorinated epoxide of formula (V):

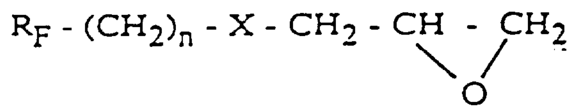 (V)

in which formulae $R_F$, $R_H$, n and x have the above meanings, X denotes O or S and Y denotes O or S, with the proviso that, when X is S, Y is not S,
in the presence of a basic or acidic compound playing the part of a reactant or catalyst; oxydation, where appropriate, of the mercaptan function to sulfoxide or sulfone with hydrogen peroxide, and recovery of the compound of formula (I) obtained.

**10.** Process according to claim 9, characterized in that the reaction of the fluorinated compound containing acidic hydrogen and the epoxide takes place in the presence or in the absence of a solvent.

**11.** Process according to claim 9 or 10, characterized in that said compound playing the part of a reactant or catalyst is present in an amount between 1 and 100 mol% relative to the fluorinated compound containing acidic hydrogen of formula (II) or (IV).

**12.** Process according to claim 11, characterized in that the amount of said compound playing the part of a reactant or catalyst is between approximately 2 and 10 mol% relative to the fluorinated compound containing acidic hydrogen of formula (II) or (IV).